# EUROPEAN PATENT APPLICATION

(11) **EP 4 621 044 A1**
(43) Date of publication of application: **24.09.2025**
(21) Application number: 23890728.1
(22) Date of filing: 13.11.2023
(51) Int. Cl.: C12N 5/071, C12N 5/075, A61K 35/12

(54) **METHOD FOR PREPARING HUMAN OVARIAN SOMATIC CELL-LIKE CELLS**

(30) Priority: 15.11.2022 CN 202211428438
(71) Applicant: Shanghai Jiao Tong University, Shanghai 200240 (CN)
(72) Inventor: WU, Ji, Shanghai 200240 (CN); GE, Junbang, Shanghai 200240 (CN); LI, Xiaoyong, Shanghai 200240 (CN)
(74) Representative: Potter Clarkson
(86) International application number: PCT/CN2023/131312
(87) International publication number: WO 2024/104294

(57) **Abstract**

A method for preparing human ovarian somatic cell-like cells, which comprises performing culturing in stages on the basis of human stem cells, where human stem cells comprise human pluripotent stem cells and human totipotent stem cells. Functional human ovarian somatic cell-like cells can be efficiently obtained, differentiation of in-vitro cultured female germline stem cells (FGSCs) is promoted, and a new avenue is provided for assisted reproduction and infertility treatment.

## Description

### FIELD OF THE DISCLOSURE

The present disclosure belongs to the field of cell biology, and more specifically, relates to a method for differentiating human pluripotent stem cells into human ovarian somatic cell-like cells.

### BACKGROUND OF THE DISCLOSURE

Infertility significantly impacts the quality of life and marital stability of millions of families, also affecting the psychological health of patients and their relatives, making it one of the major health and societal issues. The etiology of infertility involves multifactorial contributors, including nutritional deficiencies, psychological stress, organic pathologies such as tumors in the anterior pituitary, ovaries or hypothalamus, and endocrine disorders.

FGSC has a variety of clinical application values, comprising the treatment of female infertility, preservation of female fertility and delay of female menopause. In previous work, the inventors were the first internationally to discover the presence of FGSC in postnatal mouse ovaries. Using a C-terminal DDX4 antibody, germ cells were magnetically sorted from postnatal mouse ovaries, and FGSC cell line was successfully established by in-vitro culture. Transplantation of FGSC into the ovaries of infertile mice resulted in the generation of functional oocytes and offspring (Zou, K., Z. Yuan, Z. Yang, et al., Production of offspring from a germline stem cell line derived from neonatal ovaries. Nat Cell Biol, 2009. 11(5): p. 631-636). Subsequently, White et al. used the same antibody to flow sort human FGSC from the ovaries of reproductive-age women (White, Y.A.R, D.C. Woods, Y.Takai, et al., Oocyte formation by mitotically active germ cells purified from ovaries of reproductive-age women. Nat Med, 2012. 18 (3): p. 413-421). More and more studies have found that FGSC exist in mouse, rat and human ovaries. Clinical studies have found that injecting mitochondria of human FGSC into the patient's own eggs can improve egg quality and increase the success rate of in vitro fertilization (IVF) (Fakih, M.H., M.E. Shmoury, J. Szeptycki, et al., The AUGMENTSM Treatment: Physician Reported Outcomes of the Initial Global Patient Experience. JFIV Reprod Med Genet 2015. 3 (3): p. 154).

Human FGSC have opened up new ideas for delaying female menopause, treating female infertility and maintaining fertility.

In vitro differentiation of human FGSCs requires the support and induction of human ovarian somatic cell-like cells, but the sources of human ovarian somatic cell-like cells that can be used for in vitro differentiation of human FGSCs are extremely limited or unavailable.

Stem cells represent a heterogeneous population characterized by self-renewal and differentiation potential. Based on their differentiation capacity, stem cells are categorized into totipotent, pluripotent (multipotent) and unipotent subtypes. Despite extensive research and successful applications in inducing differentiation into various tissue cells (e.g., hepatocytes), technical challenges persist in deriving human ovarian somatic-like cells starting from stem cells.

### SUMMARY OF THE DISCLOSURE

The purpose of the present disclosure is to provide a method for differentiating human pluripotent stem cells into human ovarian somatic cell-like cells and uses thereof.

In the first aspect, the present disclosure provides a method for preparing human ovarian somatic cell-like cells, wherein the method is based on staged culture of human stem cells, comprising:
(1) cell pre-culture;
(2) treating the cells of (1) with a GSK3 inhibitor;
(3) treating the cells of (2) with B/A/W, wherein the B/A/W is BMP4, ActivinA and Wnt3a;
(4) treating the cells of (3) with Follistain, BMP4 and serum;
(5) treating the cells of (4) with Follistain and BMP4 to obtain a culture comprising human ovarian somatic cell-like cells.

In one or more embodiments, the "treatment" comprises "induction" or "induced treatment".

In one or more embodiments, the method is an in vitro/ex vivo culture method.

In one or more embodiments, after step (3), the obtained cells express mesendoderm markers such as brachyury, mixl1 and pax2.

In one or more embodiments, the human stem cells comprise human induced pluripotent stem cells (for example human induced pluripotent stem cells) and human totipotent stem cells (for example human embryonic stem cells).

In one or more embodiments, the "ovarian somatic cell-like cells" refer to cells expressing Foxl2, Cyp19a1 and Ptch1.

In one or more embodiments, cells are cultured by suspension culture in steps (1) to (3) and adherent culture in steps (4) to (5).

In one or more embodiments, in step (1), cells are cultured in a basal medium; in steps (2) to (5), cells are cultured in a basal medium added with GSK3 inhibitor, B/A/W, Follistain, BMP4 and serum; preferably, the basal medium is a stem cell medium (a culture medium suitable for culturing stem cells); more preferably, the basal culture medium is E8, mTesr or DMEM/F12 basal culture medium; more preferably, the basal culture medium is a basal culture medium added with 20±10% KSR, 1±0.5% NEAA, 1±0.5% Glutamax, 0.7±0.2 β-mercaptoethanol and 4-10ng/ml bFGF.

In one or more embodiments, in step (1), the time of cell pre-culture is 1 to 4 days; preferably 1.5 to 3 days; more preferably 1.5 to 2.5 days (for example 2 days).

In one or more embodiments, the culture medium for suspension culture is a liquid culture medium (culture solution). The culture medium for adherent culture is a corresponding liquid culture medium (culture solution).

In one or more embodiments, during suspension culture, low-adhesion culture dishes are used for culture.

In one or more embodiments, during adherent culture, the cells are attached to a culture dish treated with gelatin; preferably, the dish is treated with 0.2% gelatin.

In one or more embodiments, E8 is Essential 8^{™} medium from GIBCO, and mTeSR1 is a medium from Stemcell, Inc. Both culture media can be used for culturing pluripotent stem cells.

In one or more embodiments, in step (2), the GSK3 inhibitor comprises the group selected from: CHIR99021, LiCl, BIO or Ly2090314; preferably CHIR99021; preferably the concentration of CHIR99021 is 3 to 8 uM (such as 4, 4.5, 5, 5.5, 6 or 7 uM), more preferably 4 to 6 uM.

In one or more embodiments, in step (2), the culture is lasted for 30 to 48 hours (such as 32, 34, 36, 38, 40, 42 or 44 hours).

In one or more embodiments, in step (3), the concentration of BMP4 is 4-15 ng/ml (such as 5, 6, 7, 8, 9, 10, 11, 12, 13 or 14 ng/ml), preferably 9-12 ng/ml.

In one or more embodiments, the concentration of Activin A is 5-15 ng/ml (such as 6, 7, 8, 9, 10, 11, 12, 13 or 14 ng/ml), preferably 9-12 ng/ml. In one or more embodiments, the concentration of Wnt3a is 5-15 ng/ml (such as 6, 7, 8, 9, 10, 11, 12, 13 or 14 ng/ml), preferably 9-12 ng/ml. In one or more embodiments, in step (3), the culture is lasted for 2 to 9 days (such as 2.5, 3, 4, 5, 6, 7 or 8 days), preferably 2.5 to 7 days; more preferably 3 to 6 days.

In one or more embodiments, in step (4), the concentration of Follistain is 15-40 ng/ml (such as 18, 20, 22, 25, 28, 30, 32 or 35 ng/ml), preferably 20-30 ng/ml, more preferably 22-28 ng/ml.

In one or more embodiments, in step (4), the concentration of BMP4 is 4-15 ng/ml (such as 5, 6, 7, 8, 9, 10, 11, 12, 13 or 14 ng/ml), preferably 9-12 ng/ml.

In one or more embodiments, in step (4), the concentration of serum is 3-10% (v/v) (preferably 4-8%, such as 5%, 6%, 7% (v/v)).

In one or more embodiments, in step (4), the culture is lasted for 3 to 12 hours (such as 4, 5, 6, 7, 8, 9, 10 or 11 hours), preferably 4 to 10 hours; more preferably 5 to 8 hours.

In one or more embodiments, in step (5), the concentration of Follistain is 15-40 ng/ml (such as 18, 20, 22, 25, 28, 30, 32 or 35 ng/ml), preferably 20-30 ng/ml, more preferably 22-28 ng/ml. In one or more embodiments, in step (5), the concentration of BMP4 is 4-15 ng/ml (such as 5, 6, 7, 8, 9, 10, 11, 12, 13 or 14 ng/ml), preferably 9-12 ng/ml.

In one or more embodiments, in step (5), the culture is lasted for 4 to 12 days (such as 4.5, 5, 6, 7, 8, 9, 10 or 11 days), preferably 5 to 10 days; more preferably 6 to 8 days.

In another aspect, the present disclosure provides a use of any method described above for preparing human ovarian somatic cell-like cells based on staged culture of human stem cells.

In another aspect, the present disclosure provides human ovarian somatic cell-like cells (comprising cell culture) obtained by human stem cell induction and prepared by any method described above; preferably, they have characteristics selected from the following group: the human ovarian somatic cell-like cells are mainly epithelial-like, with fibroblastic cells appearing in a short spindle or triangular shape; the human ovarian somatic cell-like cells are positive for Foxl2, cyp19a1 and Ptch1 markers (including gene/transcript levels and protein levels);; and/or the human ovarian somatic cell-like cells can promote the differentiation of human female reproductive stem cells.

In another aspect, the present disclosure provides a use of human ovarian somatic cell-like cells (comprising cell culture) for promoting the differentiation of human female reproductive stem cells into human oocytes.

In another aspect, the present disclosure provides a method for promoting the differentiation of human female reproductive stem cells, wherein the method comprises: using the human ovarian somatic cell-like cells as supporting cells, culturing human female reproductive stem cells on a cell layer of the human ovarian somatic cell-like cells or mixing human female reproductive stem cells with the human ovarian somatic cell-like cells for culture.

In another aspect, the present disclosure provides a kit for preparing human ovarian somatic cell-like cells in vitro, wherein it comprises:
culture medium 1: comprising a basal culture medium and a GSK3 inhibitor; preferably, the GSK3 inhibitor is selected from: CHIR99021, LiCl, BIO or Ly2090314, more preferably CHIR99021; preferably, when used for preparing functional human ovarian somatic cell-like cells in vitro, the CHIR99021 is at a concentration of 3 to 8 uM, more preferably 4 to 6 uM in the culture system;
culture medium 2: comprising a basal culture medium and B/A/W, wherein the B/A/W is BMP4, Activin A and Wnt3a or a mixture thereof; preferably, when used for preparing functional human ovarian somatic cell-like cells in vitro, the BMP4 is at a concentration of 4 to 15 ng/ml, more preferably 9 to 12 ng/ml in the culture system, the Activin A is at a concentration of 5 to 15 ng/ml, preferably 9 to 12 ng/ml in the culture system, and the Wnt3a is at a concentration of 5 to 15 ng/ml, preferably 9 to 12 ng/ml in the culture system;culture medium 3: comprising a basic culture medium, Follistain, BMP4 and serum; preferably, when used for preparing functional human ovarian somatic cell-like cells in vitro, the Follistain is at a concentration of 15 to 40 ng/ml, preferably 20 to 30 ng/ml, more preferably 22 to 28 ng/ml in the culture system, the BMP4 is at a concentration of 4 to 15 ng/ml, preferably 9 to 12 ng/ml in the culture system, and the serum is at a concentration of 3 to 10% (v/v) in the culture system;culture medium 4: comprising a basal culture medium, Follistain and BMP4; preferably, when used for preparing functional human ovarian somatic cell-like cells in vitro, the Follistain is at a concentration of 15-40 ng/ml, preferably 20-30 ng/ml, more preferably 22-28 ng/ml in the culture system, and the BMP4 is at a concentration of 4-15 ng/ml, preferably 9-12 ng/ml in the culture system.In another aspect, the present disclosure provides a use of the kit for preparing human ovarian somatic cell-like cells based on staged culture of human stem cells.

Other aspects of the present disclosure will be apparent to those skilled in the art based on the disclosure herein.

### DESCRIPTION OF DRAWINGS

Figure 1. Bright field image of human pluripotent stem cells differentiating into human ovarian somatic cell-like cells.
Figure 2. RT-PCR detection of cell markers at various stages of cell differentiation, with GAPDH as an internal reference.
Figure 3. Comparison of Brachyury gene expression in different methods (5C: SuM Chir99021; B: BMP4; A: Activin A; W: Wnt3a).
Figure 4. Quantitative analysis results of PCR products (Group 5C) showed that the human ovarian somatic cell-like cells (OSLC) obtained by this differentiation method expressed Foxl2, Cyp19a1 and Ptch1 at high levels.
Figure 5. Identification of hPSC-derived human ovarian somatic cell-like cells using immunocytochemistry.
Figure 6. Determination of hormone secretion of hPSC-derived human ovarian somatic cells. After 48 hours of treatment with 50ug/L testosterone, the supernatant of hPSC-derived human ovarian somatic cells was collected for ELISA to measure estrogen. D14.5 is the time of inducing human ovarian somatic cells to the fifth stage of culture, that is, the 14.5th day of the entire induction differentiation process; D0 is the cells before the start of induction.
Figure 7. Human ovarian somatic cell-like cells induce differentiation of human female germ stem cells; immunofluorescence detection of the expression of GDF9 and ZP3 in cells (oocytes) formed by female germ stem cells after induction by human ovarian somatic cell-like cells.
Figure 8. RT-qPCR detection of the expression of oocyte marker genes c-KIT, FIGLA, GJA4, GDF9 and ZP3 in cells (oocytes) formed by female germ stem cells induced by human ovarian somatic cell-like cells; +OSLC, female germ cells induced by human ovarian somatic cell-like cells; Control, female germ cells induced without adding human ovarian somatic cell-like cells; *, P < 0.05, **, P < 0.01, ***, P < 0.001.

### DETAILED DESCRIPTION

Based on in-depth researches, the inventors have established a method for preparing human ovarian somatic cell-like cells in vitro, wherein it comprises performing culturing in stages on the basis of human stem cells or human totipotent stem cells. By the method of the present disclosure, functional human ovarian somatic cell-like cells can be efficiently obtained, differentiation of in-vitro cultured female germline stem cells (FGSCs) is promoted, and a new avenue is provided for assisted reproduction and infertility treatment.

### Terms

As used herein, the term "cell culture medium" in the present disclosure is a "basal culture medium", that is, a culture medium generally suitable for cell (stem cell) culture and can provide cells (stem cells) with sufficient nutrients to meet the requirements of cell growth.

As used herein, the "female reproductive stem cells" or "ovarian reproductive stem cells" are used interchangeably, and they may be cells of natural origin or cells expanded/passaged based on natural cells. In addition, they may also be cells that have been genetically engineered or modified.

As used herein, the "ovarian somatic cell-like cells" and "ovarian somatic cells" are used interchangeably, and they can be cells of natural origin or cells expanded/passaged based on natural cells, or they can also be cells induced. In addition, they can also be cells that have been genetically engineered or modified.

As used herein, human ovarian somatic cell-like cells refer to "ovarian somatic cell-like cells" that positively express Fox12, Cyp19a1 and Ptch1, also known as "ovarian somatic cells".

As used herein, "functional" means that the human ovarian somatic cell-like cells obtained according to the described method have the same or similar functions as expected.

As used herein, "differentiation" refers to the developmental process of lineage commitment. "Lineage" refers to the pathway of cell development.

### Culture and induction methods

The present disclosure is devoted to the study of in vitro large-scale preparation of functional human ovarian somatic cell-like cells, and discloses a method for differentiating human ovarian somatic cell-like cells from stem cells (comprising human pluripotent stem cells or human embryonic stem cells, preferably human pluripotent stem cells). The method utilizes the culture system of human pluripotent stem cells, adds key factors for inducing differentiation, and induces differentiation of pluripotent stem cells in vitro, thereby obtaining functional human ovarian somatic cell-like cells.

Therefore, the present disclosure provides a method for preparing human ovarian somatic cell-like cells in vitro, comprising: (1) cell pre-culture; (2) treating the cells of (1) with a GSK3 inhibitor; (3) treating the cells of (2) with B/A/W, wherein the B/A/W is BMP4, Activin A and Wnt3a; (4) treating the cells of (3) with Follistain, BMP4 and serum; (5) treating the cells of (4) with Follistain and BMP4, to obtain a culture comprising human ovarian somatic cell-like cells. During the culture, fresh culture medium can also be replaced according to the culture conditions.

The human embryonic stem cells or human stem cells involved in the present disclosure can be purchased through commercial channels, and embryonic stem cells or pluripotent stem cells may not necessarily be required by destroying embryos. As early as 1998, human embryonic stem cells and embryonic germ cells had been successfully established. For example, in 1998, the team led by Thomson finally established 5 human embryonic stem cell lines from 14 blastocysts: H1, H13, H14, H7 and H9; the team led by Gearhart isolated primitive stem cells from the gonadal ridges and mesentery of aborted fetuses aged 5-9 weeks, in order to avoid the ethical troubles caused by the direct use of embryos. See Chao Lan et al., "Research Progress of Human Embryonic Stem Cells", Advances in Modern Obstetrics and Gynecology, Volume 12, No. 4, July 2003. Based on the above work, in February 2000, the Wisconsin Alumni Research Foundation (WARF) established WiCell, an unofficial, non-profit subsidiary providing low-cost access to human embryonic stem cells for qualified scientists. There are many other institutions that provide ready-made human embryonic stem cells.

In 2006, Takahashi et al. selected four effective factors (Oct4, Sox2, c-Myc and K1f4) from 24 transcription factors and transferred them into mouse fetal or adult MEF cells using retrovirus, thereby reversing the "life clock" and successfully transforming them into induced pluripotent stem cells (iPSCs). The induced pluripotent stem cells can be cells from the body itself or pluripotent stem cells that have been expanded, cultured, passaged, or established in the field.

In the present disclosure, the female reproductive stem cells can be female reproductive stem cells from the body, or they can be female reproductive stem cells that have been expanded, cultured, passaged or established in the art. For example, the inventors have established a female reproductive stem cell line in previous studies. For example, a method for culturing (expanding) female reproductive stem cells comprises: placing female reproductive stem cells in an embryonic fibroblast (STO cell) feeder layer, and culturing them in a cell culture medium comprising sodium pyruvate, L-glutamine, mercaptoethanol, non-essential amino acids, epidermal growth factor, human basic fibroblast growth factor, glial cell growth factor and leukemia inhibitory factor. This method can culture female reproductive stem cells, but will not induce them to further differentiate into oocytes.

In the method of the present disclosure, suspension culture is used for cell induction in the early stage of induction, while adherent culture is used in the later stage. Unless otherwise specified, the culture medium used for culture or induction is a liquid culture medium (culture fluid).

In a specific embodiment of the present disclosure, a specific method for inducing differentiation of human stem cells is provided, comprising the following steps:
First, human pluripotent stem cells were cultured in suspension in a low-adhesion culture dish for 2 days to obtain embryoid bodies.
Based on the previous step, mesendoderm differentiation is performed, including treatment with an agonist of the WNT signaling pathway (GSK3 inhibitor, such as CHIR99021) for 30 to 48 hours;
Based on the previous step, Wnt3a, Activin A and BMP4 were added for 3 to 6 days;
Based on the previous step, the cells were attached to a culture dish treated with 0.2% gelatin;
Based on the previous step, Follistain, BMP4, and serum were added and treated for 5 to 8 hours.

Based on the previous step, Follistain and BMP4 were added and treated for 6 to 8 days.

In the above specific embodiment, the concentration of CHIR99021 is 5uM, the concentration of BMP is 5-10ng/ml, the concentration of Activin A is 6-10 ng/ml, the concentration of Follistain is 25 ng/ml and the concentration of WNT3A is 6-10 ng/ml.

The results tested by the inventors show that the obtained mesendoderm cells can express brachyury, mixl1 and pax2; and the obtained human ovarian somatic cell-like cells can express foxl2, cyp19a1 and ptch1.

The induced human ovarian somatic cell-like cells were identified by reverse transcription polymerase chain reaction and immunocytochemistry, and they can efficiently promote the differentiation of human female germ stem cells into oocytes.

The method of the present disclosure utilizes human stem cells to simulate the developmental laws of human ovarian somatic cells, first differentiating them into early mesoderm cells in vitro, and then continuing to induce differentiation into human ovarian somatic cell-like cells. Several main features and advantages of the method in the present disclosure are as follows: First, the entire culture system uses cell growth factors and chemical molecules, and does not introduce exogenous genes involving reprogramming or trans-differentiation functions (without changing the genome structure), thereby avoiding the interference of exogenous genes on the genome stability of the original stem cells and the potential safety hazards of transplantation related to exogenous cells. Second, human ovarian somatic cell-like cells can be efficiently obtained, and have the function of secreting estrogen and progesterone. Third, the efficiently obtained human ovarian somatic cell-like cells have an ideal cell state and can well support the differentiation of female reproductive stem cells into oocytes.

The human ovarian somatic cell-like cells obtained by the present disclosure can preferably be used as supporting cells for female reproductive stem cells to promote the efficient differentiation of female reproductive stem cells into human oocytes.

The human ovarian somatic cell-like cells obtained by the present disclosure can be introduced into the ovarian tissue of the body for necessary treatment or adjuvant therapy. For example, administration in the body comprises, but is not limited to, local injection (e.g., catheter administration or direct intraovarian injection), systemic injection, intravenous injection, intrauterine injection, and parenteral administration. The recipients are, for example (but not limited to), subjects who have difficulty conceiving, are undergoing infertility treatment, have been treated for cancer, and have undergone cytotoxic therapy (e.g., chemotherapy or radiotherapy) or a combination thereof.

The method and product of the present disclosure can not only provide an excellent model for studying the molecular mechanism of human ovarian somatic cell occurrence, but also provide a new approach for the clinical application of human ovarian somatic cells, and can be applied to assisted reproduction and infertility treatment.

### Culture medium/kit

The present disclosure also provides culture media for induction culture at each stage, including the culture media 1 to 4 described above.

In addition to the specific cytokines or chemical components listed in the embodiments of the present disclosure, cytokines or chemical components known in the art that have the same or similar functions as them can also be applied to the present disclosure. The analogs, proteins with the same functions (such as proteins with the same functions of growth factors) or compounds of the specifically listed components, equivalent compounds, analogs, derivatives and/or their salts, hydrates or precursors that induce the same target can also be used to replace the above-mentioned specific components to achieve the same technical effects. These analogs, proteins with the same functions or compounds should also be included in the present disclosure. Analogs of compounds comprise, but are not limited to: isomers and racemates of compounds. Compounds possess one or more asymmetric centers. Thus, these compounds may exist as racemic mixtures, individual enantiomers, individual diastereoisomers, diastereomeric mixtures, cis or trans isomers. The "precursor of a compound" refers to a compound that can be converted into a compound of any of the above compounds, or a salt or solution composed of a compound of any of the above compounds in the culture medium after being applied or treated by an appropriate method.

As a preferred embodiment of the present disclosure, the culture medium may further be added with ingredients for preventing bacterial contamination of cell culture, such as contamination by Gram-positive and Gram-negative bacteria, such as some antibiotics.

The cell culture medium (basal culture medium) may be, for example, but not limited to: DMEM/F12, MEM, DMEM, RPMI1640, Neuronal basal, etc. It should be understood that those skilled in the art are familiar with the preparation or purchase of the basal cell culture medium. The preferred cell culture medium is provided in the examples of the present disclosure.

The present disclosure also provides a kit, comprising the culture medium 1 to culture medium 4 described in the present disclosure; preferably, it also comprises pluripotent stem cells. Preferably, if necessary, the kit also comprises a culture medium/reagent for isolating and maintaining cells. Preferably, the kit also comprises an instruction for use, so as to facilitate the use of the kit by those skilled in the art in research or clinical application.

The disclosure is further described below in conjunction with specific embodiments. It should be understood that these examples are merely for illustrative purposes rather than intended to limit the scope of the disclosure. The experimental methods without specifying the specific conditions in the following examples generally used the conventional conditions, such as those described in J. Sambrook, Molecular Cloning: A Laboratory Manual (3rd ed. Science Press) or followed the manufacturer's recommendation.

### Example 1. Human pluripotent stem cells differentiating into human ovarian somatic cell-like cells

In this example, human ovarian somatic cell-like cells were prepared using human pluripotent stem cells as the basic cells.

### The first stage

Human induced pluripotent stem cells (iPS, obtained from Shanghai Oriental Hospital (Oriental Hospital affiliated to Tongji University) National Stem Cell Transformation Resource Bank) were suspended and cultured in low-adhesion culture bottles, with an inoculation volume of 5 x 10⁵ cells/bottle. DMEM, 20% KSR, 1% NEAA, 1% Glutamax, 0.7% β-mercaptoethanol (Sigma) were used as the basic culture medium. The culture conditions were: suspension culture at constant temperature of 37°C with 5% CO₂. The cells were cultured for 2 days to form embryoid bodies. The cells were placed for 5 minutes to allow the embryoid bodies to settle naturally. This part of the cell sphere can be used for subsequent culture.

In addition, at this stage, human induced pluripotent stem cells can also be replaced by human embryonic stem cells (H9-ES, purchased from Shanghai Chuanqiu Biotechnology Co., Ltd.).

### The second stage

Fresh culture medium was replaced for the second stage of culture.

During this stage, the inventors successively adopted two culture methods.

### (1) Method 1 (B/A/W)

B/A/W was added to the basal medium DMEM/F12 (comprising 20% KSR, 1% NEAA, 1% Glutamax, 0.7% β-mercaptoethanol), that is, the following was added:
BMP4: BMP4 with a final concentration of 5ng/mL;
Activin A with a final concentration of 6 ng/mL; and
Wnt3a with a final concentration of 6 ng/mL;
The culture is lasted for 24 hours.

### (1) Method 2 (5C)

The agonist of WNT signaling pathway, GSK3 inhibitor CHIR99021, was added to the basal culture medium DMEM/F12 (comprising 20% KSR, 1% NEAA, 1% Glutamax, 0.7% β-mercaptoethanol) at an amount of 5 uM. The culture is lasted for 36 hours.

Other culture conditions of the above methods 1 and 2: suspension culture at a constant temperature of 37°C and 5 %CO₂.

The cells were placed to allow the cell sphere to settle naturally. This part of the cell sphere can be used for subsequent culture.

### The third stage

Fresh culture medium was replaced for the third stage of culture.

Wnt3a (with a final concentration of 6 ng/ml), Activin A (with a final concentration of 6 ng/ml) and BMP4 (with a final concentration of 10 ng/ml) were added to the basal culture medium DMEM/F12 (comprising 20% KSR, 1% NEAA, 1% Glutamax, and 0.7% β-mercaptoethanol) and treated for 3 days.

The cells were placed to allow the cell sphere to settle naturally. This part of the cell sphere can be used for subsequent culture.

### The fourth stage

Fresh culture medium was replaced for the fourth stage of culture.

Based on the previous step, the cells were attached to a culture dish treated with 0.2% gelatin; Follistain-like protein 1 (Follistain or Fstl1) (with a final concentration of 25 ng/ml), BMP4 (with a final concentration of 10 ng/ml), and FBS (with a final concentration of 5%) were added to the basal culture medium DMEM/F12 (comprising 20% KSR, 1% NEAA, 1% Glutamax, and 0.7% β-mercaptoethanol) for 5-8 h.

### The fifth stage

Fresh culture medium was replaced for the fifth stage of culture.

Follistain (25 ng/ml) and BMP4 (10 ng/ml) were added to the basal medium DMEM/F12 (comprising 20% KSR, 1% NEAA, 1% Glutamax, and 0.7% β-mercaptoethanol) and treated for 8 days; no serum was added.

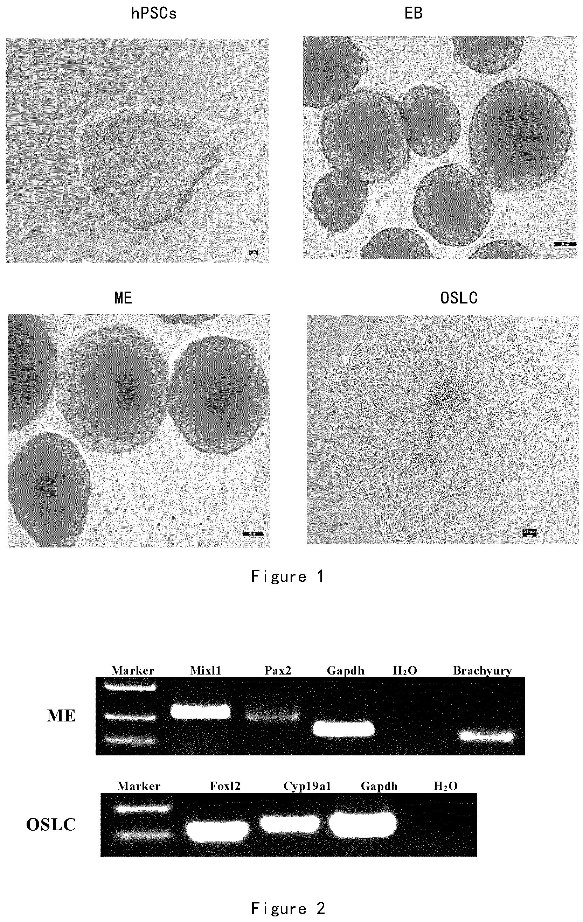

The bright field image of human ovarian somatic cell-like cells differentiated from human pluripotent stem cells obtained by the above method is shown in Figure 1. hPSCs are human pluripotent stem cells, EBs are embryoid bodies, MEs are mesendoderm cells and OSLCs are human ovarian somatic cell-like cells. It can be seen from the figure that the present disclosure obtains human ovarian somatic cell-like cells in an ideal state and in large quantities.

### Example 2. Detection of cell markers at various stages of cell differentiation

In this example, the cell markers at each stage of differentiation of cells (induced by group 5C) were detected by reverse transcription polymerase chain reaction (RT-PCR).

The RT-PCR method is as follows:
(1) Total RNA extraction
   (a) The collected cells were transferred (cells at various stages of cell differentiation) into 1 ml of Trizol, mixed by repeated pipette for 3-5 times, and placed at room temperature for 5 minutes. After placed, 200ul of chloroform was added, mixed vigorously up and down and left and right for 20 seconds, and placed at room temperature for 5 minutes;
   (b) Centrifugation at 12000 rpm for 15 min at 4°C;
   (c) The supernatant was collected into another new enzyme-free centrifuge tube, with isopropanol added in an amount equal to that of the supernatant, mixed well by up and down, and placed at room temperature for 10 min;
   (d) Centrifugation at 12000 rpm for 10min at 4°C;
   (e) The supernatant was discarded, with 1 ml of 70% anhydrous ethanol added, mixed by shaking, and centrifuged at 7600 rpm for 5 min at 4°C;
   (f) The supernatant was discarded, dried at room temperature for 2-5 min, with an appropriate amount of enzyme-free water added and mixed well. The RNA concentration and purity were measured using a nucleic acid quantifier.
(2) Reverse transcription to prepare cDNA
   According to the RNA concentration determined above and the requirements of the Hifair III 1^{st} strand Synthesis SuperMix for qPCR (Cat. No.: 11141ES10) kit, 500 ng of total RNA was taken for reverse transcription.
(3) PCR

According to the primer sequences in Table 1 and 2× Hieff^{®}PCR Master Mix (With Dye) (Cat. No. 10102ES03) kit was required for PCR.

**Table 1**

| Gene | Primer sequences |
|---|---|
| Brachyury | F: tgcttccctgagacccagtt (SEQ ID NO: 1) |
| | R: gatcacttctttcctttgcatcaag(SEQ ID NO: 2) |
| Mix11 | F: gctttcagttaccctcccagataac(SEQ ID NO: 3) |
| | R: gcacaggaagtacaataacaagtgc(SEQ ID NO: 4) |
| Pax2 | F: aacgacagaacccgactatg(SEQ ID NO: 5) |
| | R: atcccactgggtcattggag(SEQ ID NO: 6) |
| Fox12 | F: gagaagaggctcacgctgtc(SEQ ID NO: 7) |
| | R: tgatgaagcactcgttgagg(SEQ ID NO: 8) |
| Cyp19a1 | F: ggtgagagagacataaagattg(SEQ ID NO: 9) |
| | R: ttcaggataatgtttgtccc(SEQ ID NO: 10) |
| Gapdh | F: acatcaagaaggtggtgaagcagg(SEQ ID NO: 11) |
| | R: agcttgacaaagtggtcgttgagg(SEQ ID NO: 12) |
| PTCH1 | F: gaagaaggtgctaatgtcctgac(SEQ ID NO: 13) |
| | R: gtcccagactgtaatttcgcc(SEQ ID NO: 14) |

The electrophoresis results of the PCR products are shown in Figure 2. The mesendoderm cells (ME) obtained by the differentiation method can express Brachyury (Bra), Mixl1 and Pax2 (Figure 2). The human ovarian somatic cell-like cells (OSLC) obtained by the differentiation method can express Foxl2 and Cyp19a1 (Figure 2), wherein they are typical markers of human ovarian somatic cell-like cells.

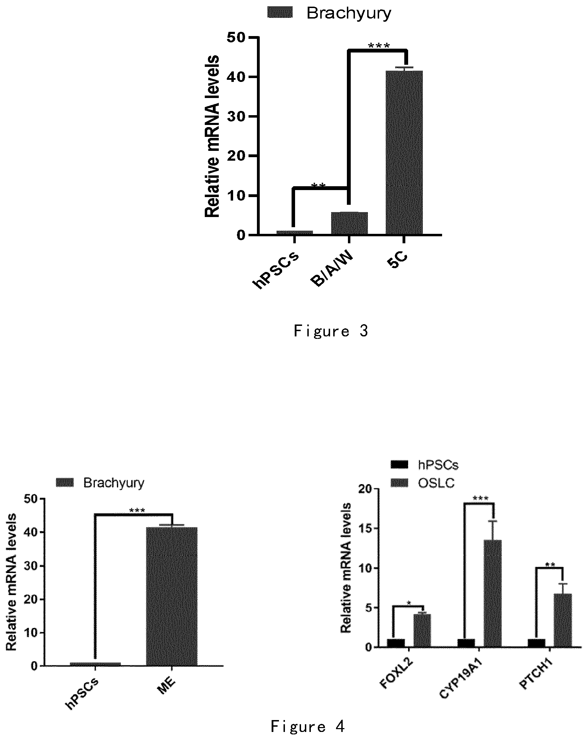

Quantitative analysis results of PCR products (Figure 4) showed that the human ovarian somatic cell-like cells (OSLC) obtained by this differentiation method expressed Foxl2, Cyp19a1 and Ptch1 at high levels.

For "Method 1" (B/A/W) and "Method 2" (5C) in the second stage of Example 1, the inventors used PCR to analyze the Brachyury marker in the cell cultures after the two methods, and the results are shown in Figure 3. As can be seen from Figure 3, the induction of 5C is more efficient in obtaining mesendoderm cells, and significantly more mesendoderm cells can be obtained, and the cell state is ideal.

### Example 3. Identification of hPSC-derived human ovarian somatic cell-like cells

In this example. Immunocytochemistry was used for identification of hPSC-derived human ovarian somatic cell-like cells.

The method of immunocytochemistry is as follows:
(a) Cells were fixed with 4% paraformaldehyde (PFA) and placed at room temperature for 30 min;
(b) The fixative solution was discarded and the cells was washed three times with calcium- and magnesium-free PBS, 5 min each time;
(c) The cells were blocked with blocking solution (PBS solution comprising 1% BSA + 0.2% Triton-X 100) for 1 h;
(d) Antibodies (Fox12, diluted 1:100; Cyp19A1, diluted 1:100; Ptch1, diluted 1:100) were added to antibody diluent (PBS solution comprising 5% BSA + 0.2% Triton-X 100);
(e) The blocking solution was aspirated and discarded; the diluted primary antibody was added to the sample and incubated at 4°C overnight;
(f) The next day, the primary antibody was discarded and the cells were washed three times with PBS, each time for 5 min;
(g) The secondary antibody (anti-goat IgG) was diluted in antibody diluent (PBS solution comprising 5% BSA);
(h) PBS was aspirated and discarded, and the diluted secondary antibody (1:200) was added to the sample and incubated at room temperature for 1 h;
(i) The secondary antibody was aspirated, discarded and the cells were washed with PBS three times, 5 min each time;
(j) DAPI solution (10 µg/ml) was added dropwise and placed at room temperature for 2-3 min;
(k) The DAPI solution was aspirated, discarded and then the cells were washed with PBS three times, 5 min each time;
(l) PBS was aspirated and discarded. The slides were sealed with anti-fluorescence quencher, observed and photographed under an inverted fluorescence microscope.

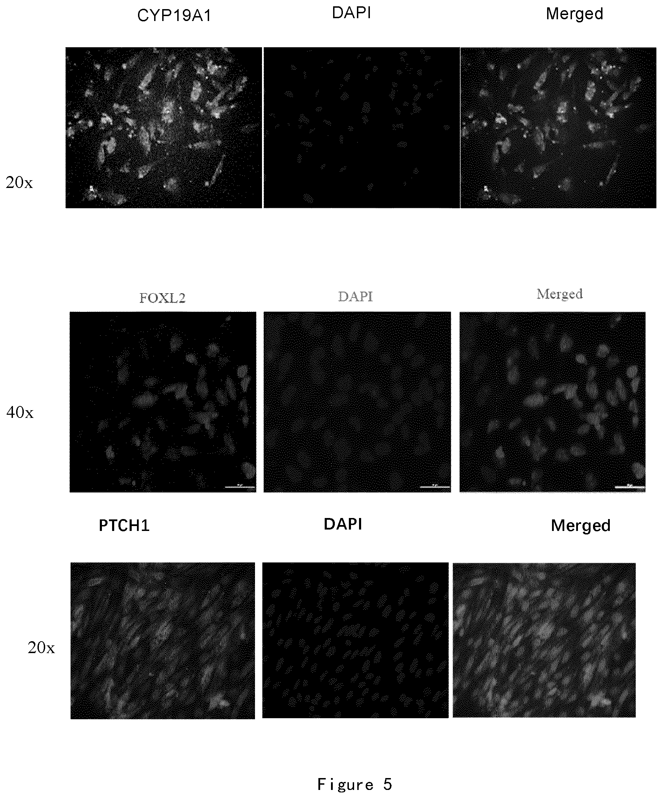

As shown in Figure 5, hPSC-derived human ovarian somatic cell-like cells express Cyp19a1, Foxl2 and Ptch1 proteins.

Regarding the methods of suspension culture and adherent culture, the present inventors have found that it is preferable to adopt suspension culture in the first to third stages and then adherent culture.

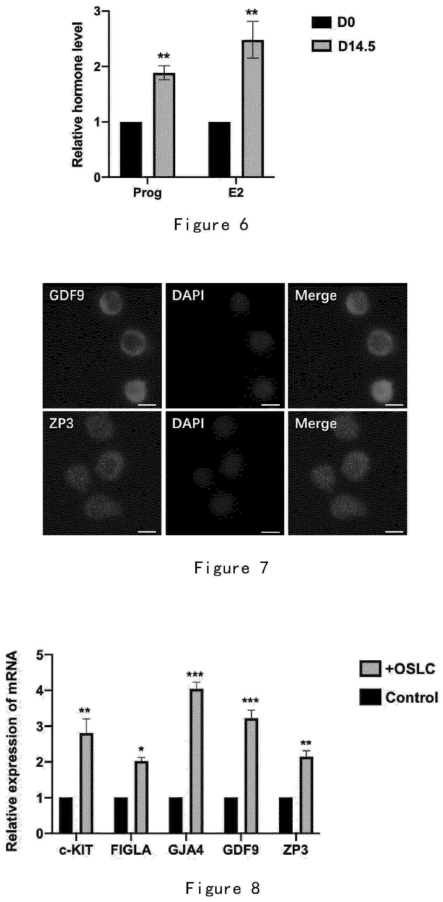

At the same time, the inventors determined the hormone secretion of hPSC-derived human ovarian somatic cells (induced by group 5C). After 48 hours of treatment with 50ug/L testosterone (added starting from day 6 of the fifth stage), the hPSC-derived human ovarian somatic cells were collected for ELISA to measure estrogen. The results showed that the human ovarian somatic cells of the present disclosure had high levels of progesterone (prog) and estradiol (E2) (Figure 6).

### Example 4. Human ovarian somatic cell-like cells promote the differentiation of female germ stem cells cultured in vitro

In this example, the inventors detected the effect of the human ovarian somatic cell-like cells induced above on the differentiation of female germ stem cells cultured in vitro.

First, the inventors inoculated human female germ stem cells onto induced human ovarian somatic cell-like cells (induced by group 5C) for culture, with a differentiation system without adding human ovarian somatic cell-like cells as a control.

Next, human female reproductive stem cells were inoculated into a human ovarian somatic cell layer and cultured for 5 days in the culture medium comprising bFGF, EGF, insulin, transferrin and RA by the inventors. Subsequently, immunofluorescence chemical analysis was used by the inventors to detect the expression of marker proteins in oocytes. The results showed that the human oocytes produced by the above method expressed oocyte-specific proteins GDF9 and ZP3, as shown in Figure 7.

At the same time, the expression of oocyte marker genes c-KIT, FIGLA, GJA4, GDF9 and ZP3 in human oocytes produced by the above method was detected using RT-qPCR by the inventors. The results showed that these marker genes were significantly upregulated, as shown in Figure 8.

These results indicate that the induced human ovarian somatic cell-like cells have the ability to promote the differentiation of female germ stem cells, with excellent effects and ideal state of induced products.

The hormones secreted by the human ovarian somatic cell-like cells produced by the above method were identified and it was found that they can secrete estrogen and progesterone and can effectively promote the differentiation of human female reproductive stem cells.

Above-described examples only show several embodiments of the present disclosure, which are described specifically and in detail. However, it should not be understood as a limiting patent scope of the present disclosure. It should be noted that those skilled in the art can make some adjustments and improvements without departing from the concept of the present disclosure and all these forms are within the scope of protection of the present disclosure. Therefore, the scope of patent protection in the present disclosure should be determined by the appended claims. Simultaneously, each reference provided herein is incorporated by reference to the same extent as if each reference was individually incorporated by reference.

## Claims

1. A method for preparing human ovarian somatic cell-like cells, wherein the method is based on staged culture of human stem cells, comprising:
(1) cell pre-culture;
(2) treating the cells of (1) with a GSK3 inhibitor;
(3) treating the cells of (2) with B/A/W, wherein the B/A/W is BMP4, Activin A and Wnt3a;
(4) treating the cells of (3) with Follistain, BMP4 and serum;
(5) treating the cells of (4) with Follistain and BMP4 to obtain a culture comprising human ovarian somatic cell-like cells.

2. The method according to claim 1, wherein, cells are cultured by suspension culture in steps (1) to (3) and adherent culture in steps (4) to (5).

3. The method according to claim 1, wherein in step (1), cells are cultured in a basal medium; in steps (2) to (5), cells are cultured in a basal medium added with GSK3 inhibitor, B/A/W, Follistain, BMP4 and serum; preferably, the basal medium is a stem cell medium.

4. The method according to claim 1, wherein the basal culture medium is E8, mTesr or DMEM/F12 basal culture medium; more preferably, the basal culture medium is a basal culture medium added with 20±10% KSR, 1±0.5% NEAA, 1±0.5% Glutamax, 0.7±0.2 β-mercaptoethanol and 4-10ng/ml bFGF.

5. The method according to claim 1, wherein in step (1), the time of cell pre-culture is 1 to 4 days; preferably 1.5 to 3 days; more preferably 1.5 to 2.5 days.

6. The method according to claim 1, wherein, in step (2),
the GSK3 inhibitor comprises the group selected from: CHIR99021, LiCl, BIO or Ly2090314; preferably CHIR99021; preferably the concentration of CHIR99021 is 3 to 8 uM, more preferably 4 to 6 uM; or
the culture is lasted for 30 to 48 hours.

7. The method according to claim 1, wherein, in step (3),
the concentration of BMP4 is 4-15 ng/ml, preferably 9-12 ng/ml; or
the concentration of Activin A is 5-15 ng/ml, preferably 9-12 ng/ml; or
the concentration of Wnt3a is 5-15 ng/ml, preferably 9-12 ng/ml; or
the culture is lasted for 2 to 9 days, preferably 2.5 to 7 days; more preferably 3 to 6 days.

8. The method according to claim 1, wherein, in step (4),
the concentration of Follistain is 15-40 ng/ml, preferably 20-30 ng/ml, more preferably 22-28 ng/ml; or
the concentration of BMP4 is 4-15 ng/ml, preferably 9-12 ng/ml; or
the concentration of serum is 3-10% (v/v); or
the culture is lasted for 3 to 12 hours, preferably 4 to 10 hours; more preferably 5 to 8 hours.

9. The method according to claim 1, wherein, in step (5),
the concentration of Follistain is 15-40 ng/ml, preferably 20-30 ng/ml, more preferably 22-28 ng/ml; or
the concentration of BMP4 is 4-15 ng/ml, preferably 9-12 ng/ml; or
the culture is lasted for 4 to 12 days, preferably 5 to 10 days; more preferably 6 to 8 days.

10. Human ovarian somatic cell-like cells obtained by human stem cell induction and prepared by the method according to any one of claims 1 to 9.

11. The human ovarian somatic cell-like cells obtained by human stem cell induction according to claim 10, wherein they have characteristics selected from the following group:
the human ovarian somatic cell-like cells are mainly epithelial-like, with fibroblastic cells appearing in a short spindle or triangular shape;
the human ovarian somatic cell-like cells are positive for Foxl2, cyp19a1, and Ptch1 markers; and/or
the human ovarian somatic cell-like cells can promote the differentiation of human female reproductive stem cells.

12. A use of human ovarian somatic cell-like cells according to claim 11 for promoting the differentiation of human female reproductive stem cells into human oocytes.

13. A method for promoting the differentiation of human female reproductive stem cells, wherein the method comprises: using the human ovarian somatic cell-like cells according to claim 10 as supporting cells, culturing human female reproductive stem cells on a cell layer of the human ovarian somatic cell-like cells or mixing human female reproductive stem cells with the human ovarian somatic cell-like cells for culture.

14. A kit for preparing human ovarian somatic cell-like cells in vitro, wherein it comprises:
culture medium 1: comprising a basal culture medium and a GSK3 inhibitor; preferably, the GSK3 inhibitor is selected from: CHIR99021, LiCl, BIO or Ly2090314, more preferably CHIR99021; preferably, when used for preparing functional human ovarian somatic cell-like cells in vitro, the CHIR99021 is at a concentration of 3 to 8 uM, more preferably 4 to 6 uM in the culture system;
culture medium 2: comprising a basal culture medium and B/A/W, wherein the B/A/W is BMP4, Activin A and Wnt3a or a mixture thereof; preferably, when used for preparing functional human ovarian somatic cell-like cells in vitro, the BMP4 is at a concentration of 4 to 15 ng/ml, more preferably 9 to 12 ng/ml in the culture system, the Activin A is at a concentration of 5 to 15 ng/ml, preferably 9 to 12 ng/ml in the culture system, and the Wnt3a is at a concentration of 5 to 15 ng/ml, preferably 9 to 12 ng/ml in the culture system;
culture medium 3: comprising a basic culture medium, Follistain, BMP4 and serum; preferably, when used for preparing functional human ovarian somatic cell-like cells in vitro, the Follistain is at a concentration of 15 to 40 ng/ml, preferably 20 to 30 ng/ml, more preferably 22 to 28 ng/ml in the culture system, the BMP4 is at a concentration of 4 to 15 ng/ml, preferably 9 to 12 ng/ml in the culture system, and the serum is at a concentration of 3 to 10% (v/v) in the culture system;
culture medium 4: comprising a basal culture medium, Follistain and BMP4; preferably, when used for preparing functional human ovarian somatic cell-like cells in vitro, the Follistain is at a concentration of 15-40 ng/ml, preferably 20-30 ng/ml, more preferably 22-28 ng/ml in the culture system, and the BMP4 is at a concentration of 4-15 ng/ml, preferably 9-12 ng/ml in the culture system.
